# EUROPEAN PATENT SPECIFICATION

(11) **EP 3 863 518 B1**
(45) Date of publication and mention of the grant of the patent: **30.04.2025**
(21) Application number: 19871330.7
(22) Date of filing: 07.10.2019
(51) Int. Cl.: A61B 5/22, A61H 99/00, A63B 23/03, G16H 20/30, G16H 40/63, A63B 24/00, A63B 21/055, A63B 21/045, A61H 23/02, A61H 21/00, A63B 21/05, A63B 71/00, A63B 71/06

(54) **TONGUE STRENGTHENING DEVICE AND METHOD FOR THE USE THEREOF**
ZUNGENVERSTÄRKUNGSVORRICHTUNG UND VERFAHREN ZU IHRER VERWENDUNG
DISPOSITIF DE RENFORCEMENT DE LANGUE ET SON PROCÉDÉ D'UTILISATION

(30) Priority: 09.10.2018 US 201862743156 P
(43) Date of publication of application: 18.08.2021
(73) Proprietor: Trudell Medical International Inc., London, Ontario N5V 5G4 (CA)
(72) Inventor: COULTES, Brandon, Ilderton, Ontario N0M 2A0 (CA); NOWAK, Bart, London, Ontario N5Z 3G6 (CA); LAVDAS, Michael, London, Ontario N6H 0J8 (CA); NUTTALL, Michael, London, Ontario N6A 3X2 (CA); SIEFFERT, Marcus, London, Ontario N5Z 1S7 (CA); SAKARIA, Ronak, London, Ontario N5Y 4V3 (CA); RIFANI, Andreas, London, Ontario N6C 4V3 (CA); ROADHOUSE, Cameron, London, Ontario N6H 4L2 (CA)
(74) Representative: Grünecker Patent- und Rechtsanwälte PartG mbB
(86) International application number: PCT/IB2019/058524
(87) International publication number: WO 2020/075041

(56) References cited:
- US-A1- 2012 302 924
- US-A1- 2012 302 924
- US-A1- 2013 296 751
- US-A1- 2013 296 751
- US-A1- 2018 064 989

## Description

This application claims the benefit of U.S. Provisional Application No. 62/743,156, filed October 9, 2018 and entitled "Tongue Strengthening Device and Method for the Use Thereof,"

### FIELD OF THE INVENTION

This invention relates to an tongue strengthening device and in particular a device used to simulate a bolus and/or apply a sensory stimulus to the oral cavity or oropharynx while sensing the force, pressure and/or movement of the tongue.

### BACKGROUND OF THE INVENTION

Swallowing is a complex sensorimotor function that serves the dual functions of transporting material from the mouth to the stomach while protecting the respiratory tract from foreign material. Swallowing involves four stages: the oral preparatory, oral, pharyngeal and esophageal stages. While the oral preparatory and oral stages are under voluntary control, with contributions from the cerebral cortex, the pharyngeal and esophageal stages are autonomic, being controlled by a brainstem network. The pharyngeal stage is triggered when an appropriate pattern of sensory stimulus excites sensory receptors within the oral cavity, oropharynx, and/or pharynx.

Dysphagia, or swallowing impairment, occurs in a number of common diseases and conditions including stroke, cerebral palsy, head and neck cancer, and Parkinson's disease. Dysphagia may affect any or several of the stages of swallowing. For example, a common swallowing abnormality in dysphagia is reduced, or delayed, triggering of the pharyngeal stage of swallowing. As a result, individuals with dysphagia often swallow less frequently when compared with healthy individuals. In addition, when swallowing is performed, the swallow may be slow and/or weak, thus placing the individual at risk of reduced nutritional intake or entry of foreign material into the respiratory tract.

Dysphagia also may result from a lack of saliva, called xerostomia. Xerostomia and associated swallowing impairment occurs in a number of patient diagnostic groups including persons who have undergone radiation therapy in the region of the salivary glands for treatment of cancer of the head or neck, persons with certain systemic conditions, e.g., Sjogren's syndrome, and persons taking medications that reduce salivary flow. When experiencing dysphagia following radiation therapy, patients may perceive their mouths to be even dryer than objective measures of saliva indicate. Unfortunately, the severity of dysphagia is correlated with the degree of perceived mouth dryness. Therefore, both dry mouth and the perception of dry mouth may be problems for patients who have undergone radiation therapy of the head and neck. In addition to the association between dry mouth and dysphagia, dry mouth is unpleasant to the patient, thereby reducing the quality of life. A variety of stimulus modalities have been applied in attempts to elicit or facilitate swallowing, including electrical stimulation of the pharynx, neck or laryngeal musculature, thermal stimulation of the faucial pillars, modification of diet, exercises, postural adjustments and the use of gustatory stimuli, such as a sour bolus, or combinations thereof.

Some oral devices, known for stimulating swallowing, include a bolus simulator, which may be manipulated by the user's tongue. Such devices, however, typically are not configured with any feedback mechanism for notifying the user about proper usage of the oral device. In addition, such oral devices lack the ability to measure tongue strength, or to track the movement and/or flexibility of the tongue, and to provide indicia to the user about such movement.

### SUMMARY OF THE INVENTION

The present invention is defined by the following claims, and nothing in this section should be considered to be a limitation on those claims.

### OUTLINE OF THE DISCLOSURE

In one aspect, one embodiment of a tongue strengthening device includes an intraoral bolus simulator having an exterior surface and an interior volume fillable with a fluid. The intraoral bolus simulator includes a sensor input component. An extraoral user interface is connected to the intraoral bolus. The intraoral bolus simulator is reciprocally moveable relative to the extraoral user interface in response to a movement of a user's tongue. The sensor input component is configured to detect a pressure or force applied to the intraoral bolus simulator and/or to detect movement of the intraoral bolus simulator relative to the extraoral user interface.

In another aspect, one embodiment of a method of strengthening a user's tongue includes gripping an extraoral user interface connected to an intraoral bolus simulator, inserting the intraoral bolus simulator into a mouth of a user between the user's tongue and palate, and reciprocally manipulating the intraoral bolus simulator with the user's tongue. The intraoral bolus may be manipulated, for example, up and down, side-to-side or front to back. The intraoral bolus simulator includes an exterior surface, has an interior volume fillable with a fluid, and includes a sensor input component. The method further includes sensing the movement of the intraoral bolus simulator, and/or the force applied to the intraoral bolus simulator with the user's tongue, with the sensor input component.

The various embodiments provide significant advantages over other types of treatment modalities for various swallowing impairments and strengthening of the tongue. For example and without limitation, various embodiments of the oral device may provide for multiple stimuli, including without limitation, gustatory, scent, somesthetic, thermal and auditory stimuli. In applicable embodiments, the fluid bolus may provide a more accurate simulator than solid devices, while also providing feedback to the user and/or caregiver about the movement of the user's tongue and the proper usage of the device, the relative strength of the user's tongue, and historic data about such strength and movement. The device is extremely portable and easy to use. Many embodiments provide for the user to use the device on their own, for example at home. At the same time, the device is provided with various safeguards, such as a shield and tether, which prevent the bolus simulator from being swallowed and/or blocking the patient's airway. In addition, in some embodiments, the device may be provided with means to deliver pharmaceutical and/or antiseptic agents.

The foregoing paragraphs have been provided by way of general introduction, and are not intended to limit the scope of the following claims. The presently preferred embodiments, together with further advantages, will be best understood by reference to the following detailed description taken in conjunction with the accompanying drawings.

### BRIEF DESCRIPTION OF THE DRAWINGS

Figure 1 is a front view of one embodiment of an oral device.
Figure 2 is a perspective view of one usage of the oral device shown in Figure 1.
Figure 3 is a perspective view of an alternative usage of the oral device shown in Figure 1.
Figure 4 is a perspective view of an alternative usage of the oral device shown in Figure 1.
Figure 5 is a perspective view of an alternative usage of the oral device shown in Figure 1.
Figure 6 is a front view of another embodiment of an oral device.
Figure 7 is a front view of another embodiment of an oral device.
Figure 8 is a front view of another embodiment of an oral device.
Figure 9 is a front view of another embodiment of an oral device.
Figure 10 is a front view of another embodiment of an oral device.
Figure 11 is a front view of another embodiment of an oral device.
Figure 12 is a front view of another embodiment of an oral device.
Figure 13 is a front view of another embodiment of an oral device.
Figure 14 is a front view of another embodiment of an oral device.
Figure 15 is a block diagram of an electronic module.
Figure 16 is a flow chart illustrating a force tracking algorithm.
Figure 17 is a flow chart illustrating a force and movement tracking algorithm.
Figure 18 is a schematic illustrating a computer structure for use with the oral device.
Figure 19 is a schematic illustrating a communication system for use with the oral device.

### DETAILED DESCRIPTION OF THE DRAWINGS AND THE PRESENTLY PREFERRED EMBODIMENTS

The term "lateral," "laterally," and variations thereof refer to the widthwise or side-to-side direction between the cheeks of the user. The term "longitudinal," "longitudinally," and variations thereof refer to the lengthwise direction of a component. The term "upper" or "above" refers to the vertical direction or orientation towards the roof of the mouth of a user when sitting upright, while the term "lower" or "below" refers to the vertical direction or orientation towards the ground. The term "fluid" refers to either a gas or a liquid, or combinations thereof, including liquids with particles or solids suspended therein. It should be understood that when referring to "first" and "second" herein, it should be understood that any terms modified thereby, including for example volume and amounts of fluid, may vary between many different measures, not just two defined measures, and that the volume and amount of fluid may be infinitely adjustable along a continuum, with "first" and "second" merely referring to two different measures along such a continuum of such measures. Reference is made to the entire disclosure of U.S Pub. No. 2013/0296751A1, directed to various aspects of an oral device, including the various embodiments of extraoral user interfaces, intraoral user interfaces, tethers, bolus simulators and the operation thereof.

### EXTRAORAL USER INTERFACE

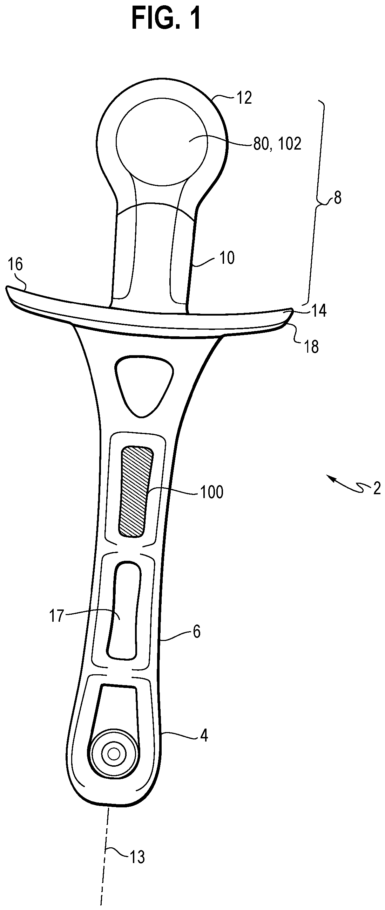

Turning now to the drawings, FIGS. 1-14, various oral devices 2 are shown as including an extraoral user interface 4. The extraoral user interface may be configured with a contoured handle 6, having various grippable features, such as ribs, knurls or other features. In various embodiments, the extraoral portions may be constructed from medical grade or other materials. For example, the extraoral portions may be made of Saint-Gobain Tygon Plastic Tubing, PolyFlav+EVA flavored plastics and/or Saline solution, 3D Systems Rapid Prototype Resin and/or Cast Urethane.

A shield 14, also forming part of the extraoral interface 4, is coupled to an intraoral member 8, configured in one embodiment with a bolus simulator 12 and tether 10. In one embodiment, the shield 14 is integrally formed as part of the extraoral interface, and is configured as a thin flange extending transversely to a longitudinal axis 13 of the handle. The shield 14 may have a slight concave contour 16 facing the bolus simulator so as to conform to the face of the user. The outer, lateral edges 18 of the shield are flared outwardly, forming a concave contour relative to a horizontal axis, and running transverse to the curved contour 16 formed about the vertical axis. The shield may be made of a hard plastic material such as polypropylene, polyethylene and/or nylon, including mineral filled or glass filled variations thereof, and may be scented, for example using a scenting agent. The shield also may be made of polycarbonate and phthalate and lead free polyvinyl chloride (PVC). In one embodiment, if more flexibility is desired, 70- to 90 Shore A durometer silicone material may be used. In one embodiment, the handle is made of polyethylene or polypropylene, while the shield is made of PolyFlav+EVA.

The shield 14 prevents the bolus simulator, or other intraoral portions, from travelling too far into the mouth cavity of the user, wherein the intraoral portion may induce a gag reflex or present a choking hazard. In addition, the shield functions as a barrier that prevents saliva or other deposits adhered to the intraoral portion from contaminating the extraoral portion, such as the handle, or the user's hand. Like the handle, it is preferable to provide the shield with aesthetic properties that avoid conjuring up an image of a device for use with infants or small children, such as a pacifier. The shield 14 may also provide a measurement device, advising as to how far the bolus simulator has been inserted into the user's mouth. In order to prevent choking in the event that the device gets dislodged in the patient's breathing air path, vent holes 15 may be incorporated into the shield.

The handle 6 may be smooth and light weight such that it does not cause injury to the oral tissues. The handle may be provided with lightening holes 17. A lanyard hole 318 may be provided at a distal end of the handle. In one embodiment, the overall weight of the oral device is less than or equal to 40 grams. The lightening holes are sized and positioned to locate the center of mass of the oral device near the proximal end of the handle to minimize the distance from the device's centroid and the center of mass, which minimizes the pendulum effect of the handle with the device is being used off-hand.

In one embodiment, the handle 6 and bolus simulator 12, as well as a tether 10 and shield 14, are permanently affixed such that they may not be separated, with the assembly being particularly well suited for a single use, single session, or for several sessions by a single user.

Alternatively, the handle 6 may be releasably connected to the bolus simulator 12 with a tether 10, for example with a clip, snap-fit or engagement with a catch member, such that the handle 6 may be separated, cleaned and reused with another bolus simulator 12.

In one embodiment, the handle may also be configured to emit a verbal cue, for example a human voice providing instructional information, for example "Get ready to swallow, swallow hard," etc. The verbal cue may be initiated manually by the user or care giver through actuation of a button interfacing with an electronic module 100 on the handle 6. Alternatively, the cue may be triggered by movement of, or pressure applied to, the bolus simulator 10 by the user.

In various embodiments, the handle may be made of the same types of materials as the shield, including a hard durometer (80 Shore A) silicone. In another embodiment, the handle includes a hard main core over molded with a softer material, such as silicone, flexible PVC or EVA with durometer values ranging from 40 Shore A to 80 Shore A.

The purpose of the handle 6 is to be used in the insertion and extraction of the intraoral part of the device from patient's mouth, as well as a means to help maneuver the bolus simulator 12 inside the patient's mouth. To serve that purpose well, the handle shall be ergonomically friendly. In addition, since in the intended treatment regiment the intaoral portion may be left inside the patient's mouth for an extended period of time, the handle 6 should be light enough that it does not have to be supported externally while the intraoral component 8 is in the patient's mouth without causing discomfort.

From an aesthetic standpoint, while the handle 6 shall retain its ease-of-use characteristics, especially for someone with impaired fine motor skills, the handle should not conjure up any negative perceptions to the intended users or patients. As an example, for patients with early onset of dementia, who are otherwise able to lead a normal social life, having to use a device that resembles something that is intended for an infant or a severely disabled person, can be quite disheartening.

Referring to FIGS. 1-15, an electronic module 100 is installed in, or connected to, the handle 6. The electronic module includes a microcontroller, memory, a power source (e.g., battery), user interfaces (e.g., buttons or switches), a display, speaker, auditory indicators (e.g., buzzers), visual interface (LCD display), bluetooth interface and/or use various visual indicators (e.g., LED). The electronic module is interfaced with various sensor input components, discussed in detail herein. The electronic module 100 records the usage of the device with a real time clock, such that the date and time of use may be recorded and stored in memory. This information may be used to monitor patient compliance and adherence to a prescribed treatment protocol. An accelerometer in the module, or other location on the handle, tether or bolus simulator, may be used to auto wake the device in response to a movement thereof. Conversely, the module may enter a sleep mode when a lack of movement over a prescribed time period is detected.

### TETHER

The oral device also includes various intraoral components 8, including a tether 10 and a bolus simulator 12. In various embodiments, the intraoral components are constructed from medical grade material. For example, the intraoral components may be made of Dow Corning Silastic M room Temperature Vulcanization (RTV) Silicone, Dow Corning Silastic MDX4 RTV Silicone, Saint-Gobain Tygon Plastic Tubing, 0.04 inch Clear Mouth Guard Thermo-Forming EVA sheets, PolyFlav+EVA flavored plastics, PVC (lead and Pthhalate free), EVA, and/or Saline solution. In one embodiment, the tether is made of a composite of fully cross-linked soft silicone outer casing and a harder silicone core. The outer casing may originate from a tubular portion of the pre-form that forms the bolus simulator, which tubular feature is compressed into a ribbon like shape in a subsequent molding process before the shield and handle components are molded thereover. The core may be an extension of the handle and shield formed during the overmolding process.

The tether may be flavored or scented by way of material impregnation, or by mechanical bonding through dipping or coating. Portions of the tether may flavored or scented, or the tether may be free of any such agents.

The tether may be made of a single material, or a composite of materials such that it satisfies applicable pull and durability tests, including but not limited to EN13450 and EN1400. Included in the pull and durability test requirements are the connection between the tether and the bolus simulator and the shield/handle. A single material design may include silicone (durometer Shore A 40 or higher), phthalate and lead free flexible PVC or EVA (durometer Shore A4 or higher). A composite material design may include reinforcing elements with a polymeric or silicone binder matrix. Various reinforcements may include woven fabrics, such as a KEVLAR material available from Du Pont, and/or tougher polymeric and silicone materials with higher durometer values than the binder. The binder material may be, but is not necessarily, the same as the overmolded layer of the shield or the casing of the malleable bolus simulator.

The tether 10 extends between the extraoral user interface 4 and the bolus simulator 12, and retains the structural integrity between those components. The tether may also be configured to communicate any external dynamic input to the bolus simulator from the operator, e.g., manipulation of the handle. In one embodiment, the tether may be configured as a thin, flat piece of material that does not substantially interfere with a closing of the user's mouth or jaws, or otherwise hinder the acts of swallowing, mastication, or chewing.

The tether 10 is flexible enough to allow for easy manipulation of the position of the bolus simulator 12 within the oral cavity, but strong enough to withstand chewing, biting, pulling etc., so as to prevent separation of the bolus simulator from the tether, and ultimately the handle or other user interface. In use, the bolus simulator may be reciprocally moveable upwardly and downwardly, side-to-side, front to back, or combinations thereof. The phrase "reciprocally moveable" refers to a back and forth movement, rather than a unitary movement where a component is moved for example against the roof of the mouth and remains there during a normal use cycle.

### BOLUS SIMULATOR

Referring to FIGS. 1-14, the intraoral bolus simulator 12 may be configured in a number of different variations. In various embodiments, the intraoral bolus simulator is configured with a core 80, which may be a solid core, a fluid (gas or liquid) filled core, whether or a constant or variable volume, or combinations thereof. In various embodiments, the gas may be air, oxygen, nitrogen, or other suitable and non-toxic gases. The fluid may be water or saline solution, and may include solid particles to provide additional texture. The core may be configured with a polymer, foam, fluid, foam gel, gel or combinations thereof in a polymeric pouch, which may present a tough but pliable characteristic.

Various gustatory stimuli may be suitable for use with the device. The outer coating, or the inner core, may be coated or impregnated with a number of chemicals known to stimulate, facilitate or evoke swallowing by means of stimulating saliva, or by way of exciting gustatory sensory endings that impinge on the brainstem or cortical swallowing networks, or by exciting other sensory nerves that are involved in the triggering of swallowing. Various gustatory agents may include without limitation NaCl, sucrose, quinine or other bitter agents, or sour agents such as lemon juice. Flavouring agents may be mixed into a silicone material or by way of coating/dipping. The flavouring agents may be scent, taste or combinations thereof.

In some embodiments, the core 80 is formed as a closed volume or hydrostat, such that the fluid contained therein may not escape during use such that it cannot be swallowed or aspirated. If the fluid is a liquid, the properties of the liquid, including the viscosity, may be varied to simulate a variety of bolus types, including without limitation a thin liquid, a thick liquid, a honey thick liquid, a puree, a fine chopped mixture, etc. The fluid may be a non-newtonian fluid. The malleable core, such as a liquid or gel, may be encased in a durable but flexible skin or pouch. The fluid filled bolus simulator 12 allows the user to manipulate the bolus simulator shape much like a masticated piece of real food, and provides an organic feel, which may aid in inducing swallowing and be manipulated to simulate swallowing. The flexible tether 10, with a minimum thickness, further provides for maximum maneuverability of the bolus simulator. The core may be composed of saline, edible and nonperishable oil, silicone gels such as SILPURAN and ELASTOSIL series gels available from Wacker, and/or propylene glycol. If both the pouch and core are made of silicone, it may be possible to vulcanize both materials together. The bolus simulator should meet the same strength and durability requirements as outlined for the tether, with additional burst resistance requirements if made from any of the materials other than the vulcanized silicone. The bolus simulator may be configured with a sealed volume of air, gel, liquid, silicone or foam, with the outer skin configured in one embodiment with a flavoring.

In one embodiment, a flexible silicone pouch encases a vulcanized silicone gel core 80, which is cured into a specific form such that it is not free flowing like a liquid in the pouch. This provides the advantage of avoiding a loss of material in the event of a breach to the pouch. In addition, the gel core maintains a structural integrity of the bolus simulator. At the same time, the cured shape of the core allows the pouch to flex without substantial stretching. The pouch may be made from a flexible but non-stretchable material, such as flexible PVC, or from stretchable rubber with lower tensile strengths. The bolus simulator may include indentations on one or both inferior and superior surfaces thereof. The indentations may be spherical, or otherwise shaped. The indentions may not be symmetrical, with a superior side shaped and contoured to mate with and fit against the roof of the user's mouth, while the inferior side is shaped and contoured to mate with the tongue. In other embodiments, the surfaces may be contoured differently to maximize the malleability of the bolus simulator.

In the various embodiments, the pouch, or jacket, e.g. silicone, may be permeable so as to allow the transfer of flavor from a flavored core to the outer surface of the pouch. Alternatively, the core may be dipped in a flavored solution, with the permeability of the pouch or jacket allowing the flavor to migrate into and remain within the pouch or the core, which may also retain the flavor from the agent. During use, the flavor is slowly released onto the outer surface of the pouch or jacket.

The bolus simulator may be provided with a reinforcement member, which reinforces the bolus stimulator so as to help prevent it from releasing particulates or suffer other damage, while maintaining soft and flexible properties. In one embodiment, the bolus simulator has a components made of elastomers and thermoplastics. In one embodiment, the edges of the device are protected, since such edges may be experience localized concentrated biting with full force. For example, a reinforcement member may be formed as a ring, which extends around the periphery of the bolus simulator. The reinforcement member may be co-moulded and/or mechanically attached to a softer portion of the bolus simulator, including the jacket filled for example with a gel. The reinforcement member may be made of the same family of material as the softer portion or other more rigid materials material. For example, various elastomer and/or thermoplastics may be used for the reinforcement member and the softer portion. The reinforcement member may also be made of various hard plastics or metal. The edge of the bolus simulator may be provided with smooth rounded sides or wide sides with steep slopes to impede the ability of the use to grab the reinforcement member with their teeth. The reinforcement member also is resistant to any tearing forces applied by the user to the bolus simulator.

Materials used to form the handle and shield include silicone materials with durometers around 60 to 80 Shore A, including for example Bluestar's USP Class VI qualified Silbione LSR 4370 is an example. Durometers for TPE options are similar to those of silicone. The bolus filler may be a gas, liquid, viscoelastic material or solid. Liquid contemplated could be saline or TPE oil. viscoelastic materials contemplated could be gels, gelatin, hydrogels, and silicone gels. An example of silicone gel is Wacker AG's Silpuran 2130 A/B.

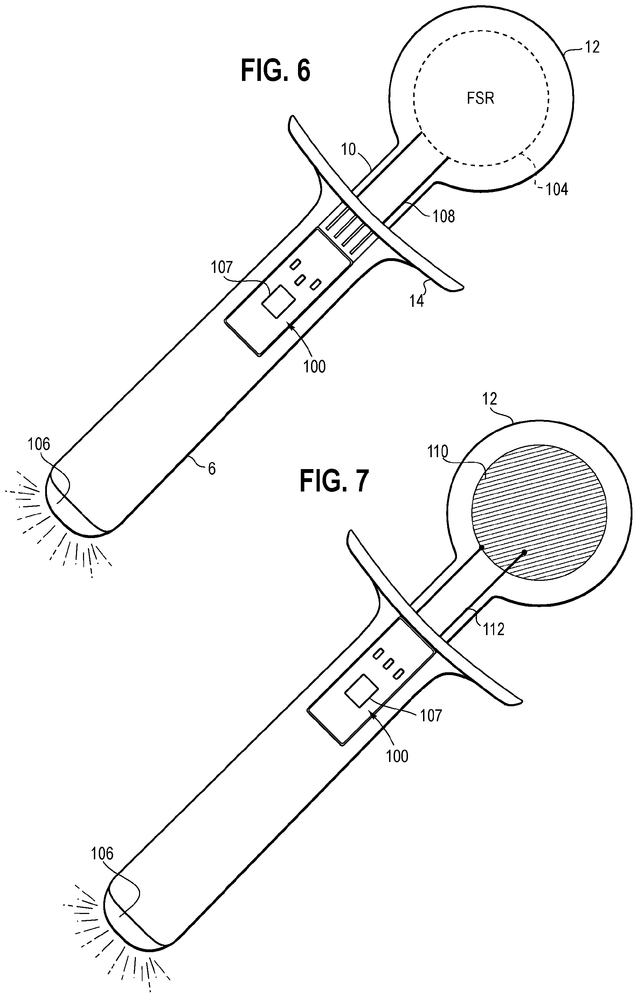

Referring to FIGS. 1 and 6-14, the bolus simulator includes a sensor input 102 component, which is configured to detect a pressure applied to the intraoral bolus simulator and/or to detect movement of the intraoral bolus simulator relative to the extraoral user interface. For example, as shown in FIG. 6, a flexible pressure/force sensor 104 may be positioned or disposed inside the bolus simulator 12, for example in an interior volume thereof. In other embodiments, it may be located on an exterior surface of the bolus simulator, including a bottom or top surface or along a periphery thereof. One suitable force sensor is the FlexiForce^{®} sensor available from Tekscan. The sensor 104 includes a resistor, which changes resistance when a pressure/force is applied. When a user applies a pressure against the bolus simulator during use, for example with their tongue as explained below, the resistance of the sensor changes, with the change measured and correlated with a tongue pressure. A use indicator 106, for example an LED coupled to the handle, may illuminate when a predetermined pressure is achieved by the user.. The sensor 104 is operably or electronically connected to the module 100 with a connector 108 that extends through the tether 10, such that a signal may be transmitted from the sensor 102 to the module 100, which may receive and store the data from the signal and determine a corresponding force value applied by the tongue for example with a microcontroller 107.

Referring to FIG. 7, the sensor input component includes a piezoelectric sensor 110, which is inserted into, or coupled to an interior or exterior of the bolus simulator 12. The sensor transforms a change in pressure, strain, or force to an electrical charge, which sends a signal to the electronic module 100 by way of a connector 112 extending through the tether 10, wherein the data may be stored in memory. When a threshold pressure is achieved, a use indicator 106, e.g., LED, is illuminated to provide feedback to the user.

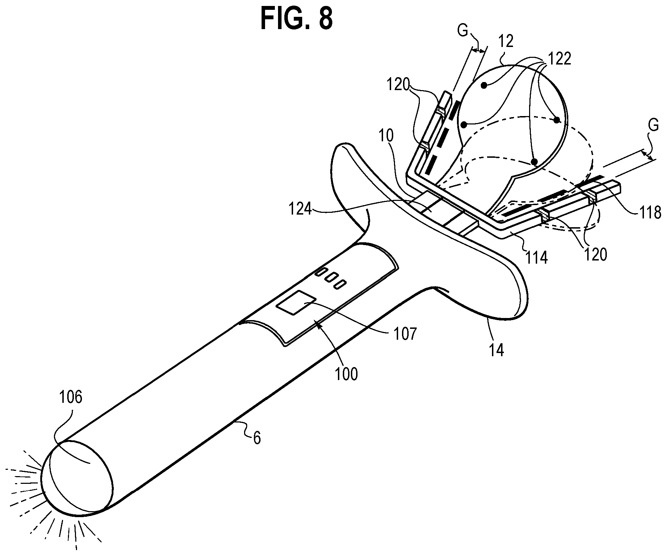

Referring to FIG. 8, the sensor input component comprises a jaw belt 114 spaced apart from the intraoral bolus simulator 12. The jaw belt may be coupled to the tether 10, the shield 14, or the extraoral user interface 4. In one embodiment, the jaw belt includes a pair of arms 116 extending along the side of the bolus simulator 12 and forming a gap (G) therebetween such that the user's teeth 118 may be received in the gap between the jaw belt and bolus simulator. Each arm may be configured with one or more hall effect sensors 120. The bolus simulator is configured with one or more magnets 122 that are positioned on or in the bolus simulator 12 so as to interface with a corresponding hall effect sensor. It should be understood that the hall effect sensors 120 may be located on the bolus simulator and the magnets 122 on the jaw belt. The hall effect sensor(s) 120 detects the proximity of a magnetic field of the magnets 122, such that when a magnetic field is applied to the hall effect sensor, the sensor turns on or off. For example, when the user moves the bolus simulator 12 side to side, or left to right, for example during an exercise regimen, the corresponding magnet 122 on the left or right side of the bolus simulator will turn the related hall sensor 120 on or off. When a threshold movement, or movement routine, is achieved, a use indicator 106, e.g., LED, is illuminated to provide feedback to the user. The hall effect sensors 120 are connected to the module with an electrical connector 124.

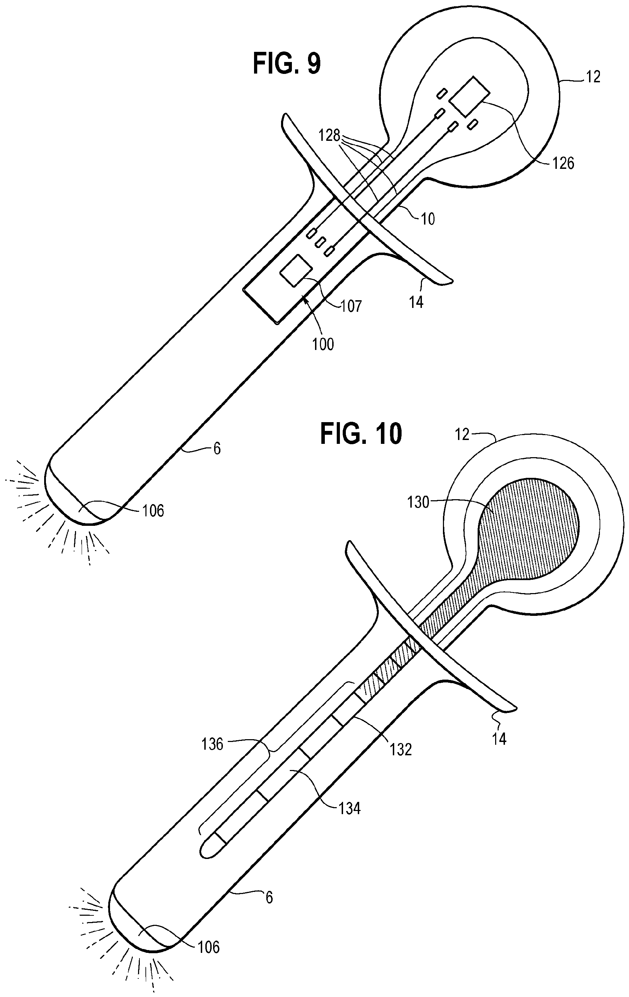

Referring to FIG. 9, the sensor input component comprises an accelerometer sensor 126 disposed in or on the bolus simulator 12. The accelerometer sensor detects movement in the X, Y and/or Z axes. When the user performs tongue movements, the accelerometer provides data output (positive or negative) about the movement based on the direction(s) of tongue movement. This data may be used to provide feedback to the user about the movement. The accelerometer 126 is electrically connected to the module 100, and microcontroller 107 with one or more electrical connectors 128.

Referring to FIG. 10, the bolus simulator 12 includes a non-newtonian fluid 130, which has characteristics of nonlinear viscosity to applied stress or pressure. A flow channel 132 extends from the bolus simulator through the tether and into the handle. The handle includes a viewing window 134 positioned over the channel such that the fluid 132 may be viewed in the channel. A gauge 136, for example including markings positioned in or on the window, or alongside the window 134, defines a use indicator in combination with the fluid 130 and viewing window 134 so as to provide feedback about the amount of pressure or force applied to the bolus simulator, as the fluid 130 is forced into the channel 132. The user may be encouraged or instructed to apply sufficient pressure or force to the bolus simulator 12 so as to move the fluid to a certain marking on the gauge. The fluid and gauge, in this way, define the sensor input component and use indicator. A switch or circuit may be arranged such that an auxiliary use indicator 106, such as an LED, provides indicia when a certain threshold pressure/force or movement is achieved.

In another embodiment, a force or pressure sensor maybe disposed in the bolus simulator with a near field communication (NFC) device or radio frequency identification (RFID) device. A NFC or RFID reader may be located in the extraoral user interface, for example in the electronic module. When a user performs an exercise, or exercise routine, the force/pressure may be transmitted from the RFID or NFC device to the reader.

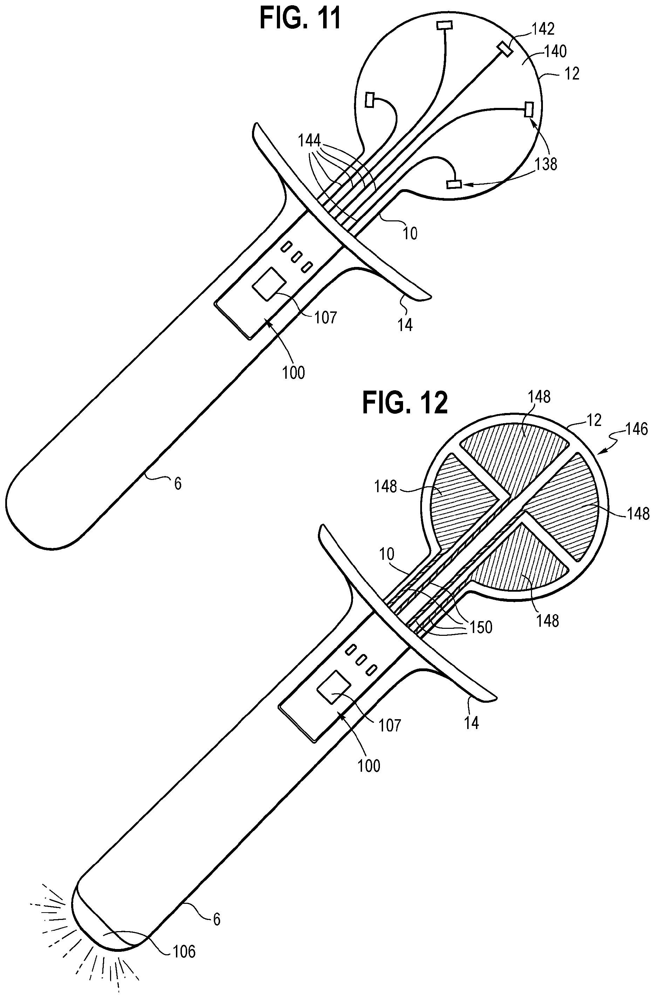

Referring to FIG. 11, an array 138 of force or pressure sensors 142 are distributed in and around the bolus simulator, for example along the outer periphery 140 thereof. The sensors measure the tongue strength and send a signal to the microcontroller 107 in the electronic module to store and analyse the readings. The array 138 of sensors 142 is electrically connected to the module 100, and microcontroller 107 with one or more electrical connectors 144.

Referring to FIG. 12, a strain gauge array 146, for example an array of four (4) strain gauges 148 arranged in the four quadrants of the bolus simulator 12, may be used to detect and measure tongue movement. The strain gauges are connected, and send signal(s), to the microcontroller in the electronic module by way of connectors 150 to store and analyse the readings.

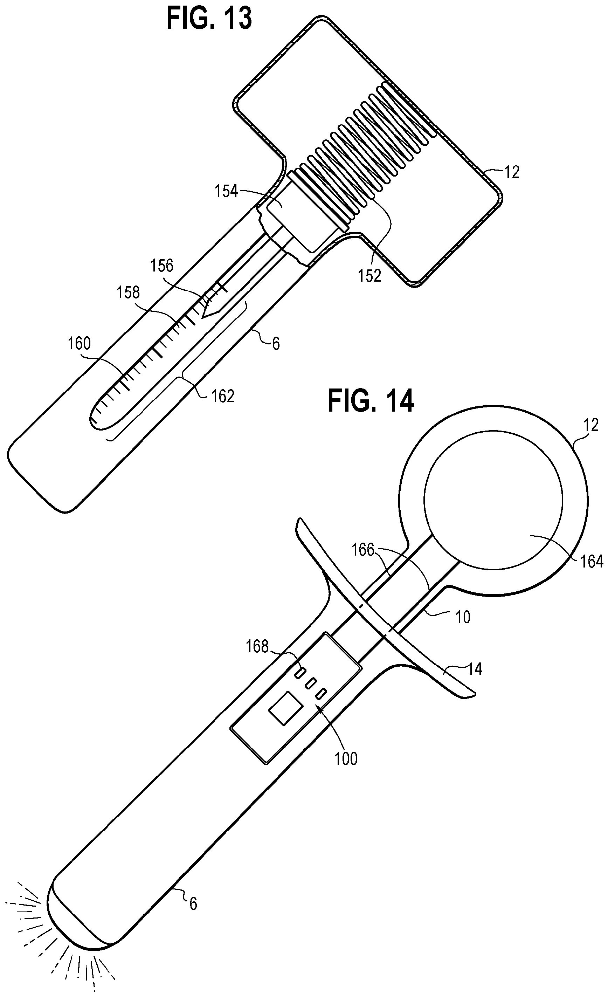

Referring to FIG. 13, a sensor input component includes a spring 152 located in the bolus simulator, with the spring acting on a plunger 154, which moves a marker 156 within a channel 158 formed in the handle 6. A viewing window 160 is disposed over the channel. A gauge 162, for example including markings positioned in or on the window, or alongside the window 160, provides feedback about the amount of pressure or force applied to the bolus simulator, as the fluid marker 156 is moved within the channel 158, with the gauge, channel and marker in combination providing a user indicator. The user may be encouraged or instructed to apply sufficient pressure or force to the bolus simulator 12 so as to move the marker 156 to a certain marking on the gauge. The spring 156, plunger 154, marker 156 and gauge 162, in this way, define the sensor input component and use indicator. A switch or circuit may be arranged such that an auxiliary use indicator 106 provides indicia when a certain threshold pressure/force or movement is achieved.

In operation, the user applied a force to the bolus simulator 12 to compress the spring 152, which moves the marker 156 to a certain level on the gauge 162. In addition to, or instead of the marker, a visual indicator, such as an LED, may provide feedback to the user that a certain force has been achieved.

Referring to FIG. 14, a vibrator 164 may be disposed in the bolus simulator 12 and connected to the electronic module 100 with electrical connectors 166. An actuator 168 on the module may be adjusted to vary the intensity of the vibrator.

### OPERATION

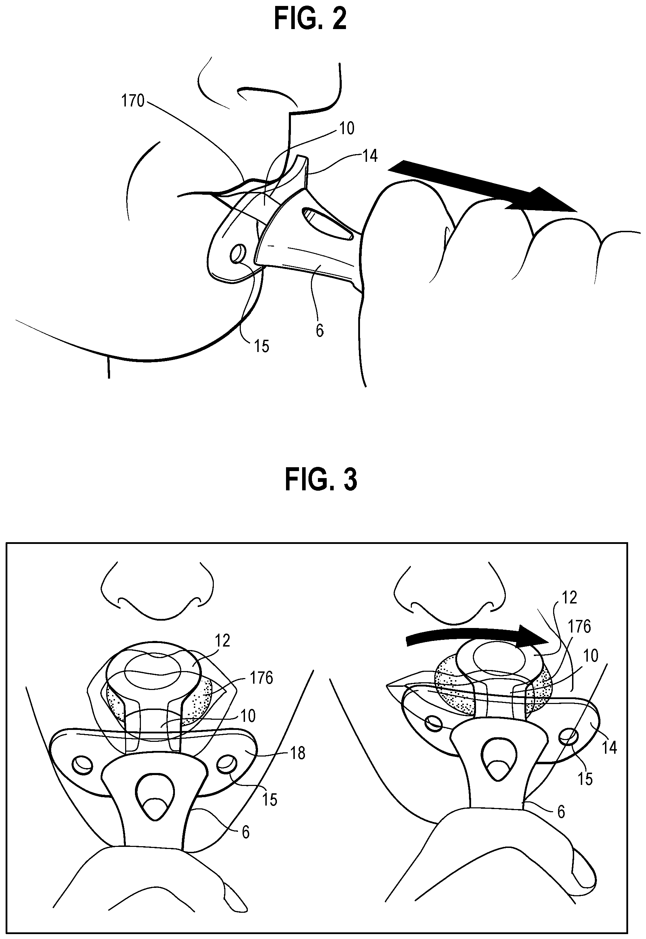

In operation, and referring to FIGS. 2-5, various exercises may be carried out by using the oral device. For example, as shown in FIG. 2, the bolus simulator 12 may be positioned in the mouth or oral cavity 174, with the user's lips 172 pressed around the tether 10. The user gently pulls on the handle 6 (e.g., with their hand) against the force of the lips. The bolus simulator may be held against the force of the lips for a predetermined time period, with the force then being relaxed and the cycle repeated.

As shown in FIG. 3, the bolus simulator 12 is positioned on the center of the tongue 176. The user reciprocally moves the bolus simulator relative to the extraoral user interface from the center to the left side of the mouth, holding it there for a predetermined time or not), and then repeats, and/or performs the same action on the right side of the mouth, or moves reciprocally left to right. The term "reciprocally," and derivatives thereof, refers to movement back and forth. The bolus simulator returns to its original position or configuration when a force is no longer applied thereto.

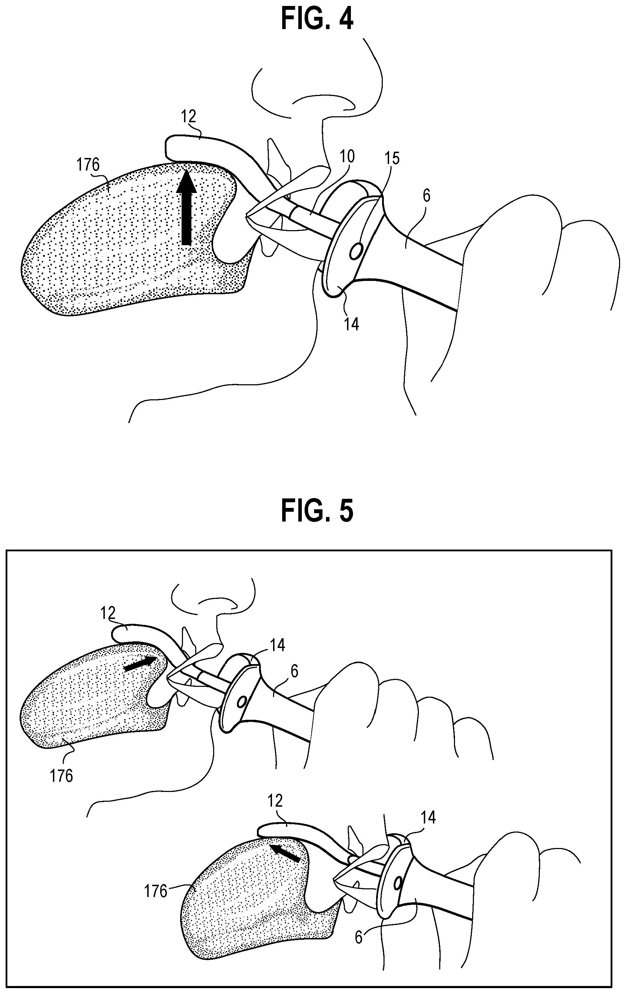

Referring to FIG. 4, the bolus simulator 12 is positioned on the center of the tongue 176. The user pushes up with their tongue 176 to move the bolus simulator upwardly relative to the extraoral user interface against the roof of their mouth, holding it there for a predetermined time period (or not), and then releasing such that the bolus simulator moves downwardly relative to the extraoral user interface. The reciprocal movement, or cycle, may be repeated.

Referring to FIG. 5, the bolus simulator 12 is positioned on the center of the tongue. The user pushes up with their tongue 176 to move the bolus simulator upwardly against the roof of their mouth, and then reciprocally moves the bolus simulator relative to the extraoral user interface forward and backward along the roof of their mouth. The oral device may also be configured with, or operably coupled to, other feedback systems, including without limitation various visual and/or oral feedback systems such as a light, scaled numeric indicia, color gradations, sound output, or combinations thereof, that are indicative of the bite or tongue force applied by the user.

The oral device also may be used to register the tongue force of the user, for example when the device is positioned on the superior surface of the tongue, or alternatively cheek force when positioned along the side of the mouth, with various biofeedback systems, including lights and sound corresponding to relative amounts of applied force. Any tongue manipulation (lateral, suck, push, pull) force may be a candidate for monitoring. The output results may also be recorded, manually or by a computer, to track progress.

In one embodiment, the user may be verbally cued (by the user, care giver or device) to prepare to swallow and then subsequently swallow. The tongue contains the bolus simulator by elevating around the bolus simulator at an anterior and lateral aspects. Due to its shape, size and position, the bolus simulator contacts the superior tongue surface, left and right lateral tongue margins and palate. In various embodiments, the bolus simulator stimulates both sides of the oral cavity/oropharynx, or only one side of the oral cavity. If pharyngeal swallowing is triggered, the tongue presses against the palate in an anterior-to-posterior direction, which may release a bolus, such as a fluid, from the inner core in some embodiments. The user then swallows the bolus. Users with upper dentures should remove the dentures prior to use to avoid any interference with stimulation of the sensory receptive fields on the palate.

The physical specifications of the bolus stimulator may stimulate the oral cranial nerve afferents. For example, when positioned on the superior surface of the tongue, the bolus simulator may stimulate a variety of sensory receptors lining the tongue surface, as well as lining the hard and soft palates. The anterior 2/3 of the tongue receives somatic sensory innervations from the trigeminal (v) nerve, and taste sensation from the facial nerve (VII), and the glossopharyngeal extends into the anterior 2/3 of the tongue, particularly along the lateral tongue margin, with anasomoses between the IX and V nerves. In this way, the bolus simulator may stimulate the V, VII and IX afferent fibers that are critical for a number of oral sensorimotor behaviors including but not limited to normal food transport, mastication, taste, swallowing, speech production and salivation.

While the bolus simulator may be positioned on the superior tongue surface and maintained in a stationary position or moved by the tongue relative to the extraoral user interface as described above, the user, or caregiver, may also move the bolus simulator within the oral cavity by manually manipulating the handle 6. For example and without limitation, the bolus simulator may be rotated on the tongue surface, or displaced to make contact with the buccal cavity, hard and soft palates, sub-lingual region, tongue surface, and anterior facial pillar, the latter of which is believed to play a role in eliciting pharyngeal swallowing. Various approaches may also include stroking the bolus simulator along the tongue surface, which may excite both gustatory and somatosensory receptors. The user may also manipulate the bolus simulator as if it were a masticated piece of food, ready to be swallowed, with the tether preventing actual swallowing of the device. The simulator can also be treated like a lollypop, with the user practicing sucking motions. Flavoring of the bolus simulator may help the user to imagine or conjure that the bolus simulator is a real piece of masticated food, with the scented shield also serving a similar function due to the position of the shield positioned under the nose.

The bolus simulator is positioned in the mouth relative to the teeth and jaw. The oral device targets the start of the pharyngeal swallow phase. The pharynx is in communication with both the esophagus and the larynx. The device also encourages the user to engage motor functions (chewing, tongue movement, etc.) that may facilitate triggering or initiation of the patterned pharyngeal swallow. The device may also be used by persons with oral preparatory stage dysfunction, such as those with cancer resection, without the oral preparatory phase. The portion of the device that extends between the teeth and lips of the user is as thin as possible to facilitate full closure of the mouth and occlusion of the teeth. At the same time, the shield may be moved and located to position the bolus simulator in the proper location in the mouth.

The deformable bolus simulator, whether variable in volume or when configured as a hydrostat, provides an opportunity for subtle movements of the tongue to be met by changes in the local sensory environment, which in turn may facilitate changes in tongue posture/position and corresponding oral sensory input. The user may participate in intensive oral sensorimotor transformations by maintaining the deformable bolus simulator on the superior surface of the tongue, and thereby simulate various properties of a food/liquid bolus to be swallowed. The device may also be used as a preventative measure, for example to maintain a strong and healthy swallow function in the elderly population, for example by helping them to maintain their ability to eat and drink.

Referring to FIG. 8, the user positions the jaw belt 114 alongside the outer surfaces of the user's teeth 118, which are disposed in the space or gap (G) between the jaw belt and bolus simulator.

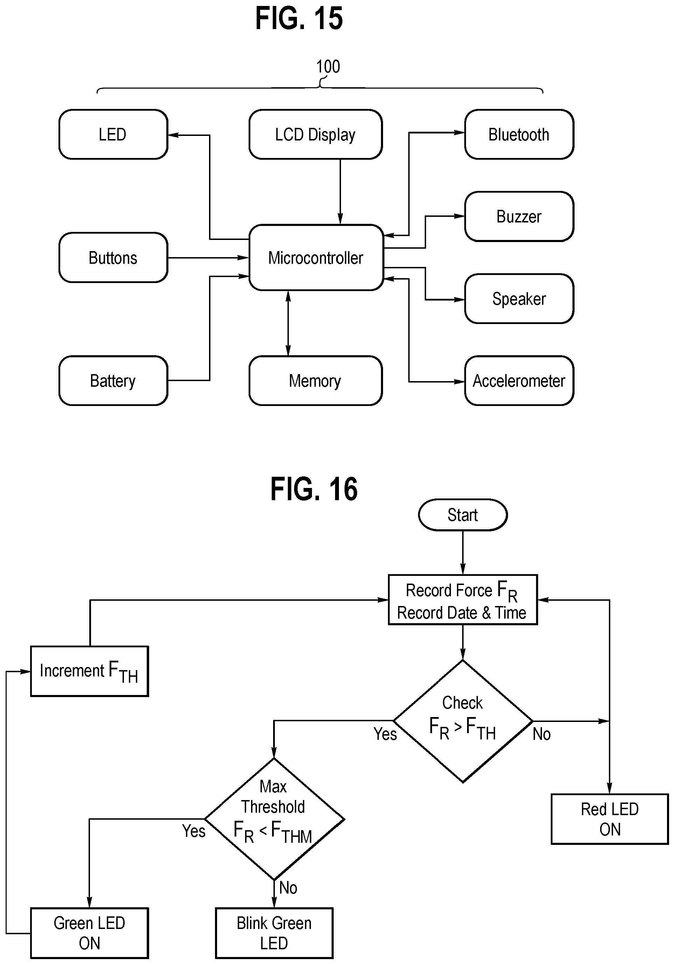

Referring to FIG. 16, feedback information on the tongue exercise and swallowing may be provided to the user using visual use indicators, such as LED(s) and LCD displays, or with auditory use indicators, such as speakers or buzzers. When the user performs an exercise, the force or pressure applied by the tongue maybe monitored by the sensor and recorded. An algorithm tracks the applied tongue force, with a threshold force representing the force of a healthy person. The user may be encouraged, through visual and auditory feedback, to each the threshold force/pressure value. Different threshold values may be set according to patient needs. The algorithm tracks the tongue force applied by the user, and compares the recorded force values with a threshold value, which may be set by the user or a caregiver, including the user's doctor. If the recorded value is higher than the threshold value, meaning the user is able to achieve the required tongue pressure/force, the recorded tongue pressure is then compared with the maximum threshold value. If the recorded value is below the maximum threshold value, then the user is notified with a green LED being illuminated. The algorithm then encourages the user to achieve higher tongue pressure/force by incrementing the threshold value set by the user or caregiver. In this way, the user can be trained to achieve a goal and regain their tongue strength. If the recorded tongue pressure is lower than the threshold value then the user is notified by illuminating a red LED, which informs the user that more pressure/force is required. In this way, the user is encouraged to achieve the minimum threshold value.

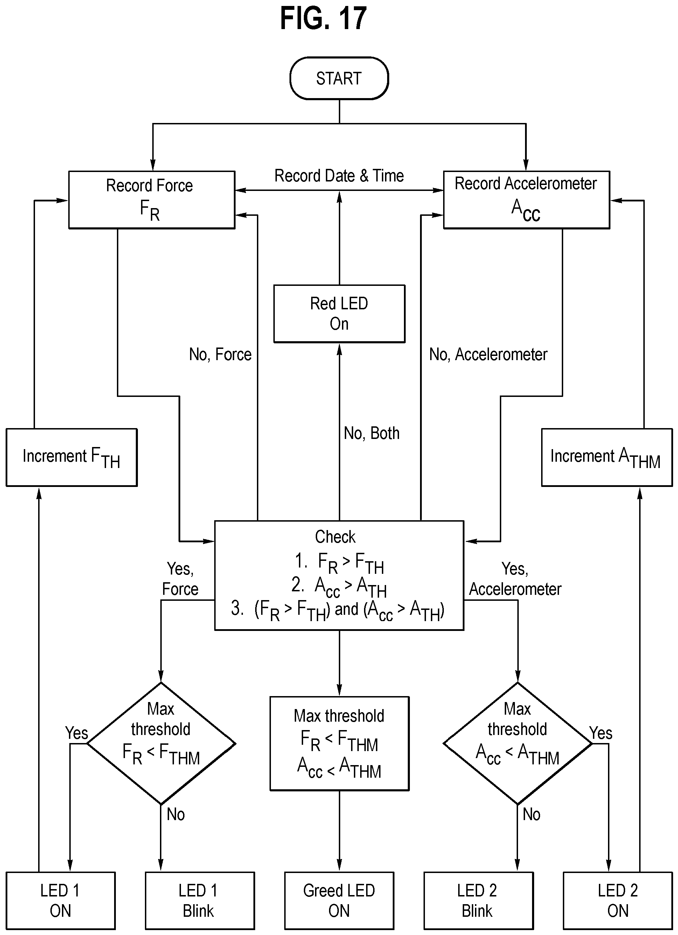

Referring to FIG. 17, various sensors, such as the disclosed accelerometer 126, can be used to track movement of the bolus simulator 12 and tongue 176. In this way, movement information, together with the force/pressure feedback, may help the user to achieve their exercise goals. Bluetooth technology may be use to transfer the recorded force and movement values to a smartphone or other computer, along with the date and time of the usage. In this type of algorithm, the force and movement of the tongue are recorded simultaneously. The force and movement are compared with threshold values. Upon achieving a required tongue force, LED 1 turns on notifying the user that the force requirement has been achieved. The threshold force value is then incremented. Likewise, recorded movement value in the X, Y and Z directions are compared to respective threshold values and maximum threshold values. Upon achieving the required movement LED 2 turns on, meaning the user has achieved the required movement. If the force and movement are both achieved relative to threshold values, then the green LED is turned on, notifying the user that the exercise has been completed. If the user fails to achieve the threshold values, the red LED is illuminated. If maximum threshold value force or movement is achieved, the LED1 and LED 2 start blinking so as to notify the user that the force or movement needs to be controller.

The data related to the tongue force/pressure and/or movement may be recorded in memory and stored. The data may be transferred using Bluetooth, or by hardwire connections, to a smartphone or other computer to track the exercise session(s). The oral device may be programmed to remind the user or caregiver to perform an exercise regimen, with an auditory or visual indicator providing the indicia.

In order to provide faster and more accurate processing of the data, for example from one or more various sensors, generated within the oral device, data may be wirelessly communicated to a smart phone, local computing device and/or remote computing device to interpret and act on the raw sensor data.

In one implementation, the electronic module includes circuitry for transmitting raw sensor data in real-time to a local device, such as a smart phone. The smart phone may display graphics or instructions to the user and implement processing software to interpret and act on the raw data. The smart phone may include software that filters and processes the raw sensor data and outputs the relevant status information contained in the raw sensor data to a display on the smart phone. The smart phone or other local computing device may alternatively use its local resources to contact a remote database or server to retrieve processing instructions or to forward the raw sensor data for remote processing and interpretation, and to receive the processed and interpreted sensor data back from the remote server for display to the user by way of a user indicator, e.g., a display or user interface, or a caregiver that is with the user of the oral device.

In addition to simply presenting data, statistics or instructions on a display of the smart phone or other local computer in proximity of the oral device, proactive operations relating to the oral device may be actively managed and controlled. For example, if the smart phone or other local computer in proximity to the oral device determines that the sensor data indicates the end of treatment has been reached, or that further treatment is needed, the smart phone or other local computing device may communicate such information directly to the user or patient. Other variations are also contemplated, for example where a remote server in communication with the smart phone, or in direct communication with the oral device via a communication network, can supply the information and instructions to the patient/user.

In yet other implementations, real-time data gathered in the oral device and relayed via to the smart phone to the remote server may trigger the remote server to track down and notify a physician or supervising caregiver regarding a problem with the particular treatment session or a pattern that has developed over time based on past treatment sessions for the particular user. Based on data from the one or more sensors in the oral device, the remote server may generate alerts to send via text, email or other electronic communication medium to the user, the user's physician or other caregiver.

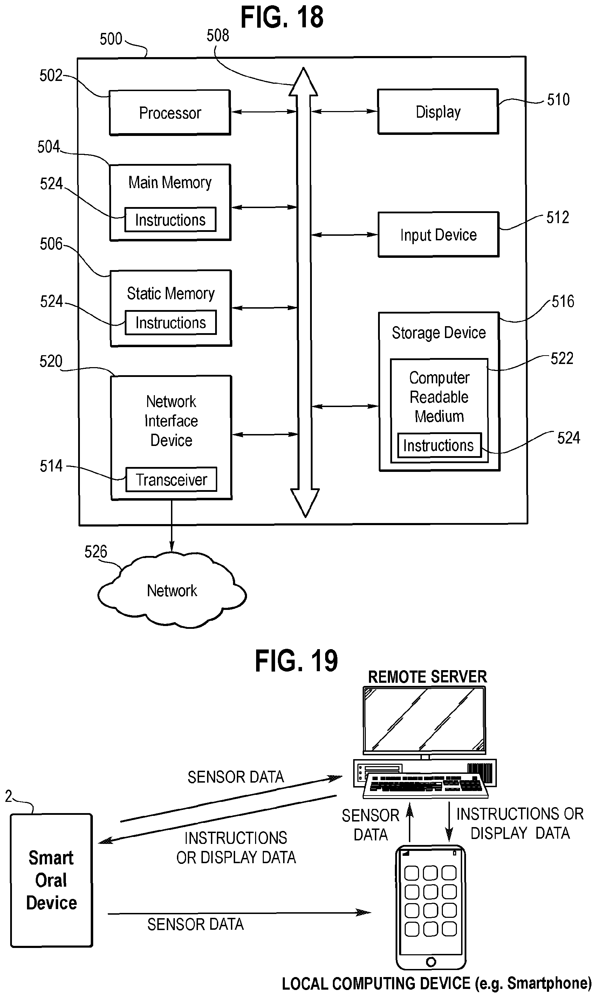

The electronic circuitry in the oral device, the local computing device (e.g. the microcontroller) and/or the remote server discussed above, may include some or all of the capabilities of a computer in communication with a network and/or directly with other computers. As illustrated in FIGS. 18 and 19, the computer 500 may include a processor 502, a storage device 516, a display or other output device 510, an input device 512, and a network interface device 520, all connected via a bus 508. A battery 503 is coupled to and powers the computer. The computer may communicate with the network. The processor 502 represents a central processing unit of any type of architecture, such as a CISC (Complex Instruction Set Computing), RISC (Reduced Instruction Set Computing), VLIW (Very Long Instruction Word), or a hybrid architecture, although any appropriate processor may be used. The processor 502 executes instructions and includes that portion of the computer 500 that controls the operation of the entire computer. Although not depicted in FIGS. 18 and 19, the processor 502 typically includes a control unit that organizes data and program storage in memory and transfers data and other information between the various parts of the computer 500. The processor 502 receives input data from the input device 512 and the network 526 reads and stores instructions (for example processor executable code) 524 and data in the main memory 504, such as random access memory (RAM), static memory 506, such as read only memory (ROM), and the storage device 516. The processor 502 may present data to a user via the output device 510.

Although the computer 500 is shown to contain only a single processor 502 and a single bus 508, the disclosed embodiment applies equally to computers that may have multiple processors and to computers that may have multiple busses with some or all performing different functions in different ways.

The storage device 516 represents one or more mechanisms for storing data. For example, the storage device 516 may include a computer readable medium 522 such as read-only memory (ROM), RAM, non-volatile storage media, optical storage media, flash memory devices, and/or other machine-readable media. In other embodiments, any appropriate type of storage device may be used. Although only one storage device 516 is shown, multiple storage devices and multiple types of storage devices may be present. Further, although the computer 500 is drawn to contain the storage device 516, it may be distributed across other computers, for example on a server.

The storage device 516 may include a controller (not shown) and a computer readable medium 522 having instructions 524 capable of being executed on the processor 502 to carry out the functions described above with reference to processing sensor data, displaying the sensor data or instructions based on the sensor data, controlling aspects of the oral device to alter its operation, or contacting third parties or other remotely located resources to provide update information to, or retrieve data from those remotely located resources. In another embodiment, some or all of the functions are carried out via hardware in lieu of a processor-based system. In one embodiment, the controller is a web browser, but in other embodiments the controller may be a database system, a file system, an electronic mail system, a media manager, an image manager, or may include any other functions capable of accessing data items. The storage device 516 may also contain additional software and data (not shown), which is not necessary to understand the invention.

The output device 510 is that part of the computer 500 that displays output to the user. The output device 510 may be a liquid crystal display (LCD) well-known in the art of computer hardware. In other embodiments, the output device 510 may be replaced with a gas or plasma-based flat-panel display or a traditional cathode-ray tube (CRT) display. In still other embodiments, any appropriate display device may be used. Although only one output device 510 is shown, in other embodiments any number of output devices of different types, or of the same type, may be present. In one embodiment, the output device 510 displays a user interface. The input device 512 may be a keyboard, mouse or other pointing device, trackball, touchpad, touch screen, keypad, microphone, voice recognition device, or any other appropriate mechanism for the user to input data to the computer 500 and manipulate the user interface previously discussed. Although only one input device 512 is shown, in another embodiment any number and type of input devices may be present.

The network interface device 520 provides connectivity from the computer 500 to the network 526 through any suitable communications protocol. The network interface device 520 sends and receives data items from the network 526 via a wireless or wired transceiver 514. The transceiver 514 may be a cellular frequency, radio frequency (RF), infrared (IR) or any of a number of known wireless or wired transmission systems capable of communicating with a network 526 or other smart devices 102. The bus 508 may represent one or more busses, e.g., USB, PCI, ISA (Industry Standard Architecture), X-Bus, EISA (Extended Industry Standard Architecture), or any other appropriate bus and/or bridge (also called a bus controller).

The computer 500 may be implemented using any suitable hardware and/or software, such as a personal computer or other electronic computing device. The computer 500 may be a portable computer, laptop, tablet or notebook computers, smart phones, PDAs, pocket computers, appliances, telephones, and mainframe computers are examples of other possible configurations of the computer 500. The network 526 may be any suitable network and may support any appropriate protocol suitable for communication to the computer 500. In an embodiment, the network 526 may support wireless communications. In another embodiment, the network 526 may support hard-wired communications, such as a telephone line or cable. In another embodiment, the network 526 may support the Ethernet IEEE (Institute of Electrical and Electronics Engineers) 802.3x specification. In another embodiment, the network 526 may be the Internet and may support IP (Internet Protocol). In another embodiment, the network 526 may be a LAN or a WAN. In another embodiment, the network 526 may be a hotspot service provider network. In another embodiment, the network 526 may be an intranet. In another embodiment, the network 526 may be a GPRS (General Packet Radio Service) network. In another embodiment, the network 526 may be any appropriate cellular data network or cell-based radio network technology. In another embodiment, the network 526 may be an IEEE 802.11 wireless network. In still another embodiment, the network 526 may be any suitable network or combination of networks. Although one network 526 is shown, in other embodiments any number of networks (of the same or different types) may be present.

It should be understood that the various techniques described herein may be implemented in connection with hardware or software or, where appropriate, with a combination of both. Thus, the methods and apparatus of the presently disclosed subject matter, or certain aspects or portions thereof, may take the form of program code (i.e., instructions) embodied in tangible media, such as floppy diskettes, CD-ROMs, hard drives, or any other machine-readable storage medium wherein, when the program code is loaded into and executed by a machine, such as a computer, the machine becomes an apparatus for practicing the presently disclosed subject matter. In the case of program code execution on programmable computers, the computing device generally includes a processor, a storage medium readable by the processor (including volatile and non-volatile memory and/or storage elements), at least one input device, and at least one output device. One or more programs may implement or use the processes described in connection with the presently disclosed subject matter, e.g., through the use of an API, reusable controls, or the like. Such programs may be implemented in a high level procedural or object-oriented programming language to communicate with a computer system. However, the program(s) can be implemented in assembly or machine language, if desired. In any case, the language may be a compiled or interpreted language and it may be combined with hardware implementations. Although exemplary embodiments may refer to using aspects of the presently disclosed subject matter in the context of one or more stand-alone computer systems, the subject matter is not so limited, but rather may be implemented in connection with any computing environment, such as a network or distributed computing environment. Still further, aspects of the presently disclosed subject matter may be implemented in or across a plurality of processing chips or devices, and storage may similarly be spread across a plurality of devices. Such devices might include personal computers, network servers, and handheld devices, for example.

Providing feedback to users regarding their technique is one feature of the oral device that will help optimize treatment. A controller, which may be located on or inside the various embodiments of the oral device described herein, is in communication with one or more sensors, switches and or gauges that are tracking or controlling operation of the oral device. The controller may store data gathered in a memory for later download to a receiving device, or may transmit data to a receiving device in real-time. Additionally, the controller may perform some processing of the gathered data from the sensors, or it may store and transmit raw data. RF transmitter and/or receiver modules may be associated with the controller on the oral device to communicate with remote hand-held or fixed computing devices in real-time or at a later time when the oral device is in communication range of a communication network to the remote hand-held or fixed location computing devices. The controller may include one or more of the features of the computer system 500 shown in FIG. 83. Additionally, the one or more sensors, switches or gauges may be in wired or wireless communication with the controller.

For clarity in displaying other features of the various oral device embodiments described, the controller circuitry is omitted from some illustrations, however a controller or other processing agent capable of at least managing the routing or storing of data from the oral device is contemplated in one version of these embodiments. In other implementations, the oral device may not include an onboard processor and the various sensors, gauges and switches of a particular embodiment may wirelessly communicate directly with a remotely located controller or other processing device, such as a handheld device or remote server. Data gathered by a controller or other processing device may be compared to expected or pre-programmed values in the local controller memory or other remote location to provide the basis for feedback on whether desired performance or therapy is taking place. If the controller is a more sophisticated and includes more of the computer 500 elements described in FIG. 18, then this processing may all be local to the oral device. In more rudimentary controller arrangements, the data may simply be date/time stamped and stored locally or remotely for later processing. In one embodiment, the data may further be locally or remotely stamped with a unique device or patient identifier.

## Claims

1. A tongue strengthening device comprising:
an intraoral bolus simulator (12) comprising an exterior surface and having an interior volume fillable with a fluid,
wherein the intraoral bolus simulator comprises a sensor input component; and
an extra oral user interface (4) connected to the intraoral bolus simulator, wherein the intraoral bolus simulator is reciprocally moveable relative to the extra oral user interface in response to a movement of a user' s tongue, and wherein the sensor input component is configured to detect a pressure or force applied to the intraoral bolus simulator and/or to detect movement of the intraoral bolus simulator relative to the extra oral user interface, wherein the extra oral user interface comprises a use indicator (106) configured to provide feedback to the user about the pressure or force applied to the intra oral bolus simulator and/or the movement of the intra oral bolus simulator relative to the extra oral user interface,
wherein the sensor input component comprises an array (138) of force sensors (142) disposed in the intra oral bolus simulator,
and the force sensors comprise a resistor,
**characterized in that**
the sensor input component comprises a jaw belt spaced apart from the intraoral bolus simulator, wherein one of the jaw belt and intraoral bolus simulator comprises a hall effect sensor, and wherein the other of the jaw belt and intraoral bolus simulator comprises a magnet.

2. The tongue strengthening device of claim 1 further comprising a tether (10) connecting the extra oral user interface (4) and the intraoral bolus simulator (12), wherein the tether (10) is flexible.

3. The tongue strengthening device of claim 1 wherein the intraoral bolus simulator is moveable upwardly and downwardly, side-to-side and/or front-to-back relative to the extra oral user interface in response to a movement of a user's tongue.

4. The tongue strengthening device of claim 1 wherein the use indicator comprises a LED.

5. The tongue strengthening device of claim 1 further comprising an electronics module (100) disposed in the extra oral user interface, wherein the sensor input component is operably connected to the electronics module and wherein the electronics module comprises a power source, microcontroller and memory.

6. The tongue strengthening device of claim 1 wherein the sensor input component comprises a near field communication (NFC) device or radio frequency identification (RFID) device disposed in the intraoral user interface, and a NFC or RFID reader disposed in the extra oral user interface.

## Patentansprüche

1. Zungenstärkungsvorrichtung, umfassend:
einen intraoralen Bolus-Simulator (12), der eine Außenfläche umfasst und ein Innenvolumen aufweist, welches mit einer Flüssigkeit gefüllt werden kann, wobei der intraorale Bolus-Simulator eine Sensoreingangskomponente umfasst, und
eine extraorale Benutzerschnittstelle (4), die mit dem intraoralen Bolus-Simulator verbunden ist, wobei der intraorale Bolus-Simulator in Reaktion auf eine Bewegung der Zunge eines Benutzers relativ zur extraoralen Benutzerschnittstelle wechselseitig bewegbar ist und wobei die Sensoreingangskomponente so konfiguriert ist, dass sie einen auf den intraoralen Bolus-Simulator ausgeübten Druck oder eine auf den intraoralen Bolus-Simulator ausgeübte Kraft erfasst und/oder eine Bewegung des intraoralen Bolus-Simulators relativ zur extraoralen Benutzerschnittstelle erfasst, wobei die extraorale Benutzerschnittstelle einen Verwendungsindikator (106) umfasst, welcher so konfiguriert ist, dass er dem Benutzer eine Rückmeldung über den auf den intraoralen Bolus-Simulator ausgeübten Druck oder die auf den intraoralen Bolus-Simulator ausgeübte Kraft und/oder die Bewegung des intraoralen Bolus-Simulators relativ zur extraoralen Benutzerschnittstelle zur Verfügung stellt,
wobei die Sensoreingangskomponente eine Anordnung (138) von Kraftsensoren (142), die im intraoralen Bolus-Simulator angeordnet sind, umfasst und die Kraftsensoren einen Widerstand umfassen,
**dadurch gekennzeichnet, dass** die Sensoreingangskomponente ein Kieferband umfasst, das in einem Abstand von dem intraoralen Bolus-Simulator angeordnet ist, wobei eines von dem Kieferband und dem intraoralen Bolus-Simulator einen Hall-Effekt-Sensor umfasst und wobei das andere von dem Kieferband und dem intraoralen Bolus-Simulator einen Magneten umfasst.

2. Zungenstärkungsvorrichtung nach Anspruch 1, die ferner einen Haltestreifen (10) umfasst, welcher die extraorale Benutzerschnittstelle (4) und den intraoralen Bolus-Simulator (12) verbindet, wobei der Haltesstreifen (10) flexibel ist.

3. Zungenstärkungsvorrichtung nach Anspruch 1, wobei der intraorale Bolus-Simulator in Reaktion auf eine Bewegung der Zunge eines Benutzers nach oben und unten, seitlich und/oder von vorne nach hinten relativ zur extraoralen Benutzerschnittstelle beweglich ist.

4. Zungenstärkungsvorrichtung nach Anspruch 1, wobei der Verwendungsindikator eine LED umfasst.

5. Zungenstärkungsvorrichtung nach Anspruch 1, die ferner ein Elektronikmodul (100) umfasst, welches in der extraoralen Benutzerschnittstelle angeordnet ist, wobei die Sensoreingangskomponente betriebsfähig mit dem Elektronikmodul verbunden ist und wobei das Elektronikmodul eine Stromquelle, einen Mikrocontroller und einen Speicher umfasst.

6. Zungenstärkungsvorrichtung nach Anspruch 1, wobei die Sensoreingangskomponente eine Nahfeldkommunikationsvorrichtung (NFC) oder eine Radiofrequenzidentifikationsvorrichtung (RFID), die in der intraoralen Benutzerschnittstelle angeordnet ist, und ein NFC- oder RFID-Lesegerät, das in der extraoralen Benutzerschnittstelle angeordnet ist, umfasst.

## Revendications

1. Dispositif de renforcement de langue comprenant
un simulateur de bolus intraoral (12) comprenant une surface extérieure et un volume intérieur pouvant être rempli d'un fluide,
dans lequel le simulateur de bolus intraoral comprend un composant d'entrée de capteur, et
une interface utilisateur extraorale (4) connectée au simulateur de bolus intraoral, dans laquelle le simulateur de bolus intraoral peut effectuer un mouvement de vient par rapport à l'interface utilisateur extraorale en réponse à un mouvement de la langue de l'utilisateur, et dans laquelle le composant d'entrée du capteur est configuré pour détecter une pression ou une force appliquée au simulateur de bolus intraoral et/ou pour détecter le mouvement du simulateur de bolus intraoral par rapport à l'interface utilisateur extraorale, l'interface utilisateur extraorale comprend un indicateur d'utilisation (106) configuré pour fournir à l'utilisateur un retour d'information sur la pression ou la force appliquée au simulateur de bolus intraoral et/ou sur le mouvement du simulateur de bolus intraoral par rapport à l'interface utilisateur extraorale,
dans lequel le composant d'entrée du capteur comprend un réseau (138) de capteurs de force (142) disposés dans le simulateur de bolus intraoral,
et les capteurs de force comprennent une résistance,
**caractérisé par le fait que**
le composant d'entrée du capteur comprend une courroie de mâchoire espacée du simulateur de bolus intraoral, dans laquelle l'une des courroies de mâchoire et le simulateur de bolus intraoral comprennent un capteur à effet Hall, et dans laquelle l'autre courroie de mâchoire et l'autre simulateur de bolus intraoral comprennent un aimant.

2. Dispositif de renforcement de langue selon la revendication 1, comprenant en outre un filin (10) reliant l'interface utilisateur extraorale (4) et le simulateur de bolus intraoral (12), le filin (10) étant flexible.

3. Dispositif de renforcement de langue selon la revendication 1, dans lequel le simulateur de bolus intraoral peut être déplacé vers le haut et vers le bas, d'un côté à l'autre et/ou de l'avant à l'arrière par rapport à l'interface utilisateur extraorale en réponse à un mouvement de la langue de l'utilisateur.

4. Dispositif de renforcement de langue selon la revendication 1, dans lequel l'indicateur d'utilisation comprend une DEL.

5. Dispositif de renforcement de langue selon la revendication 1 comprenant en outre un module électronique (100) disposé dans l'interface utilisateur extrabuccale, dans lequel le composant d'entrée du capteur est connecté de manière opérationnelle au module électronique et dans lequel le module électronique comprend une source d'énergie, un microdispositif de commande et une mémoire.

6. Dispositif de renforcement de langue selon la revendication 1, dans lequel le composant d'entrée du capteur comprend un dispositif de communication en champ proche (NFC) ou un dispositif d'identification par radiofréquence (RFID) disposé dans l'interface utilisateur intraorale, et un lecteur NFC ou RFID disposé dans l'interface utilisateur extraorale.
